# EUROPÄISCHE PATENTSCHRIFT

(11) **EP 0 929 807 B1**
(45) Veröffentlichungstag und Bekanntmachung des Hinweises auf die Patenterteilung: **22.01.2003**
(21) Anmeldenummer: 97942027.0
(22) Anmeldetag: 12.09.1997
(51) Int. Cl.: G01N 33/32, G01N 35/00

(54) **VERFAHREN UND VORRICHTUNG ZUR VERMESSUNG VON LACKIERTEN PRÜFTAFELN**
METHOD AND DEVICE FOR MEASURING PAINTED TEST TABLES
PROCEDE ET DISPOSITIF DE MESURE D'ECHANTILLONS PEINTS

(30) Priorität: 30.09.1996 DE 19640376
(43) Veröffentlichungstag der Anmeldung: 21.07.1999
(73) Patentinhaber: BASF Coatings Aktiengesellschaft, 48165 Münster (DE)
(72) Erfinder: BERLIN, Harald, D-48301 Nottuln (DE); BIALLAS, Bernd, D-48324 Albersloh (DE); DUSCHEK, Wolfgang, D-48165 Münster (DE); ROTZ, Werner, D-48163 Münster (DE)
(74) Vertreter: Fitzner, Uwe, Dr.
(86) Internationale Anmeldenummer: EP9705005
(87) Internationale Veröffentlichungsnummer: WO98014778

(56) Entgegenhaltungen:
- US-A- 4 561 776
- US-A- 4 615 902
- US-A- 4 920 385
- US-A- 5 062 298
- US-A- 5 550 632
- US-A- 5 619 319
- VALCARCEL ET AL: "Automatic Methods of Analysis. Techniques and Instrumentation in Analytical Chemistry Vol. 9" 1988 , ELSEVIER , AMSTERDAM NL XP002051739 siehe Seite 261 - Seite 264

## Beschreibung

Die vorliegende Erfindung betrifft ein Verfahren und eine Vorrichtung zur Vermessung von lackierten Prüftafeln.

Für die Entwicklung und Qualitätskontrolle von Lacken und Anstrichstoffen ist es erforderlich, die damit erzielbaren Beschichtungen auf verschiedene Eigenschaften hin zu untersuchen. Zu diesem Zweck werden in der Regel Probebeschichtungen hergestellt, für die sich die Bezeichnung "Prüftafel" eingebürgert hat, da der probeweise beschichtete Gegenstand häufig plattenförmig ist. Es gibt jedoch auch verschieden stark gekrümmte "Prüftafeln", z.B. wenn Teile von Autokarosserien verwendet werden oder wenn gerade die Lackeigenschaften in Krümmungszonen untersucht werden sollen.

Die mit Hilfe von Prüftafeln untersuchten Eigenschaften betreffen ein breites Spektrum relevanter Eigenschaften der Beschichtung. Hierzu gehören zum einen die optischen Eigenschaften und hierbei insbesondere die Farbeigenschaften der Beschichtung (Farbton, Glanz, Verlauf, Effekteigenschaften, Haze, Deckvermögen). Zum anderen interessieren die mechanischen Eigenschaften, wie z.B. Härte der Beschichtung, Haftung am Untergrund und Elastizität. Schließlich interessieren weitere physikalische Eigenschaften wie das Diffusionsvermögen fremder Stoffe in der Schicht, die elektrische Leitfähigkeit der Schicht, das UV-Absorptionsvermögen, die Flammschutzwirkung sowie die Beständigkeit der Schicht unter Praxisbeanspruchungen. Ja nach spezieller Aufgabe der Beschichtung können weitere relevante Parameter hinzukommen. Andererseits kann es der Fall sein, daß für einen bestimmten Lack oder Anstrichstoff nicht alle der aufgezählten Eigenschaften von besonderer Bedeutung sind, sondern daß das Augenmerk auf einige wenige gerichtet werden kann.

Bislang ist es üblich, die Untersuchung von Prüftafeln manuell mit Hilfe geeigneter Meßgeräte durchzuführen. Hierzu wird im Labor jede einzelne Prüftafel von einem Bearbeiter mit den entsprechenden Meßgeräten für die Eigenschaften untersucht und die Meßergebnisse werden in der Regel von Hand notiert für die weitere Auswertung. Dieses Vorgehen ist nicht nur sehr arbeitsaufwendig, sondern darüber hinaus auch störanfällig und mit systematischen Fehlern behaftet. So hängt die Durchführung der Messungen z.B. von den Gewohnheiten und der Geschicklichkeit des Testers ab, und aufgrund des Arbeitsaufwandes finden zu jeder Eigenschaft nur wenige Messungen, in der Regel nur eine Messung, statt. Da viele Beschichtungseigenschaften wechselseitig (funktional) voneinander abhängen, z.B. die Härte von der Schichtdicke, wäre es besonders wichtig, die Messungen möglichst unter gleichen Bedingungen, insbesondere am selben Ort auf der Prüftafel durchzuführen. Dieses ist jedoch bei dem bisherigen Vorgehen nicht gewährleistet, da bei den manuellen Tests in der Regel nicht darauf geachtet wird, an welchem Ort der Prüftafel gemessen wird, und da ein solcher Ort von Hand auch nicht mit der notwendigen Präzision reproduzierbar bestimmt werden kann. Die bisher angewendeten Meßverfahren sind somit arbeitsaufwendig und darüber hinaus mit hohen Fehlern behaftet. Schließlich kann es bei der manuellen Durchführung der Tests auch zu Verwechselungen kommen, wenn versehentlich Meßdaten den falschen Prüftafeln zugeordnet werden.

Eine automatische Messvorrichtung wird in der EP-0 383 322 A2 für biochemische Analysen beschrieben. Bei dieser werden Indikatorpräparate auf Objektträgem aus einem Vorrat erfaßt und entlang einer festgelegten Bahn durch die Messvorrichtung geführt, die sie schließlich an einer Auswurfstation verlassen. Auf ihrem Weg werden die Indikatorpräparate mit der zu untersuchenden Probenflüssigkeit versetzt, und eventuell einsetzende optische oder elektrochemische Veränderungen werden von einem entsprechenden Sensor gemessen. Eine derartige oder eine vergleichbare automatische Messvorrichtung wäre bei der Vermessung von lackierten Prüftafeln jedoch unanwendbar bzw. nachteilig, da sie ein festes geometrisches Format der Prüftafeln erfordert, nur eine einzige Art von Sensor enthält und insbesondere nicht die üblichen und vorhandenen (manuellen) Meßgeräte verwenden kann.

Die US 4 615 902 offenbart eine Vorrichtung und ein Verfahren, mit denen ein unsortierter und unvermessener Stapel von Farbprüftafeln mit unterschiedlichen Farbeigenschaften automatisch in verschiedene Farbeigenschafts-Klassen einsortiert werden kann. Die Farbtafeln sind dabei mit einem maschinenlesbaren Identifikationscode versehen. Sie werden von einem ersten Roboter nach ihrer Ablieferung in einer Ablage einzeln ergriffen und zunächst an einer Leseeinrichtung zur Bestimmung des ldentifikationscodes und dann an einem ortsfesten Messgerät zur Bestimmung einer Farbeigenschaft vorbeigeführt. Nach der Messung legt der Roboter die Prüftafeln in einem Ausgangsstapel ab. Die gewonnenen Messwerte werden in einem Computer den jeweiligen Identifikationscodes zugeordnet. Nach Abschluss der Messung kann der Computer somit alle gewonnenen Messwerte in Gruppen einteilen, wobei eine Gruppe alle im Rahmen einer vorgegebenen Toleranz als gleich zu betrachteten Prüftafeln enthält. Die vom Computer berechnete logische Einteilung der Prüftafeln wird in einem zweiten Verarbeitungsschritt von einem zweiten Roboter in eine physikalische Sortierung der Prüftafeln umgesetzt. Der zweite Roboter ergreift zu diesem Zweck jede Prüftafel, führt sie an einem Lesegerät für den Identifikationscode vorbei, und legt sie dann auf einem Stapel entsprechend der vom Computer berechneten Gruppe ab.

Aufgabe der vorliegenden Erfindung war es demgegenüber, ein Verfahren und eine Vorrichtung zur Verfügung zu stellen, die die geschilderten Nachteile nicht aufweisen. Die Vermessung von Prüftafeln soll daher erheblich vereinfacht und gleichzeitig mit einer höheren Präzision und Reproduzierbarkeit möglich sein. Das Verfahren soll ohne Schwierigkeiten flexibel zwischen verschiedenen Prüftafelgeometrien und den Messungen verschiedener Eigenschaften wechseln können und die Meßergebnisse sicher den vermessenen Prüftafeln zuordnen, wobei insbesondere auch ortsaufgelöste Messungen möglich sein sollen. Die Messungen selber sollen mit verschiedenen Meßgeräten an einer Probe ortsaufgelöst, d. h. hochpräzise Messungen am selben Ort, durchgeführt werden. Ferner sollen vorhandene (Hand-)Meßgeräte einsetzbar sein.

Diese Aufgabe wird durch ein Verfahren gelöst, welches die folgenden Schritte enthält:
a) Ablage der beschichteten Prüftafeln in einer Aufgabestation,
b) Überführung einer Prüftafel aus der Aufgabestation zu einer Meßstation durch eine Greif- und Bewegungsvorrichtung,
c) Ergreifen von Meßgeräten durch die Greif- und Bewegungsvorrichtung und Durchführung der Messungen an der Prüftafel in der Meßstation,
d) Überführung der vermessenen Prüftafel in eine Ablagestation durch die Greif- und Bewegungsvorrichtung.

Mit dem erfindungsgemäßen Verfahren verläuft die Vermessung der Prüftafeln nach deren Herstellung vollautomatisch. Die notwendigen Bearbeitungs- und Bewegungsschritte werden durch eine Greif- und Bewegungsvorrichtung ("Manipulator") durchgeführt. Diese ist insbesondere dafür geeignet, die verschiedenen Meßgeräte zu ergreifen, zur Prüftafel zu führen, die Messung an der Prüftafel durchzuführen und das Meßgerät schließlich an den ursprünglichen Ort wieder abzustellen. Dabei ist aufgrund der maschinellen Durchführung des Meßprozesses insbesondere gewährleistet, daß präzise bekannte Orte auf der Prüftafel angefahren und vermessen werden. Insbesondere kann dabei die Messung aller Eigenschaften am selben Ort stattfinden, so daß eine richtige Korrelation der Eigenschaften untereinander hergestellt wird. Beim bisherigen manuell durchgeführten Verfahren war dies nicht gewährleistet. Werden z.B. Schichthärte oder Farbdeckungsvermögen an einem anderen Ort als die Schichtdicke gemessen, so können diese Eigenschaften nicht korreliert werden und eigentlich vorhandene Wechselbeziehungen werden nicht beobachtet. Bei dem erfindungsgemäßen Verfahren werden dagegen alle Messungen an demselben Ort durchgeführt. Hierdurch steigert sich die Präzision des Resultates, da aus der Praxis oder der Theorie bekannte Wechselbeziehungen zwischen den Eigenschaften ausgenutzt werden können, um die jeweiligen Meßwerte untereinander abzugleichen. Durch die Darstellbarkeit des funktionalen Zusammenhanges zwischen verschiedenen Meßgrößen können sogar völlig neue Eigenschaften neben den bisher erfaßten Werten ermittelt werden, z.B. das Deckvermögen in der Schrägsicht oder die Wolkenbildung in der Schrägsicht. Desweiteren kann mit dem erfindungsgemäßen Verfahren die Präzision der Messung erhöht werden, indem alle Eigenschaften mehrfach, vorzugsweise an verschiedenen Orten der Prüftafel, bestimmt werden. Aufgrund der vollautomatischen Durchführung des Verfahrens sind solche Mehrfachmessungen nicht mit erhöhtem personellem Arbeitsaufwand verbunden. Bei Bedarf können derartige Messungen auch außerhalb der üblichen Arbeitszeit, z.B. nachts, durchgeführt werden. Mit Hilfe der mehrfach vorhandenen Meßergebnisse läßt sich dann zum einen eine statistische Auswertung des Resultates durchführen, die zu einer erhöhten Präzision führt, zum anderen werden die Eigenschaften mit dem Meßort auf der Prüftafel korreliert. Eine derartige ortsaufgelöste Messung liefert weitere wertvolle Hinweise über die Eigenschaften der Beschichtung der Prüftafel.
Das erfindungsgemäße Verfahren ist hinsichtlich der Zahl und Art der eingesetzten Meßgeräte äußerst flexibel, und durch eine entsprechende Programmierung der Steuerung der Greif- und Bewegungsvorrichtung können insbesondere auch die herkömmlichen Handmeßgeräte nunmehr im automatischen Betrieb verwendet werden. Darüber hinaus ist das Verfahren aber auch flexibel in bezug auf die vermessenen Prüftafeln. Diese können von unterschiedlicher Größe und Form sein, insbesondere verschiedene Krümmungen haben. Dadurch, daß die Meßgeräte von der Greif- und Bewegungsvorrichtung erfaßt und geführt werden, können sie insbesondere auch auf frei vorgebbaren Bahnen bewegt werden, so daß insbesondere Messungen entlang gekrümmter Oberflächen möglich sind.
Die freie Beweglichkeit der Greif- und Bewegungsvorrichtung hat ferner den Vorteil, daß sie eine kompakte Anordnung der Meßgeräte und Prüfobjekte ermöglicht.

In einer Weiterentwicklung des erfindungsgemäßen Verfahrens werden die mit Hilfe der Meßgeräte gewonnenen Meßdaten elektronisch erfaßt, gespeichert, ausgewertet und dargestellt. Die elektronische Erfassung der Meßdaten in einer zentralen Einheit erleichtert erheblich die Übersicht und Weiterverarbeitung der erhaltenen Daten.

Die automatische Verarbeitung der Prüftafeln kann ferner dadurch abgerundet werden, daß
a) die Prüftafel vor ihrem Einbringen in die Aufgabestation mit einer maschinenlesbaren Kennzeichnung versehen werden, welche Informationen zur Identifizierung und damit des Prüfplans enthält,
b) die maschinenlesbare Kennzeichnung automatisch erfaßt wird und die darin enthaltenen Informationen in die Steuerung der Greif- und Bewegungsvorrichtung und/oder der Datenerfassung übernommen werden.

Bei der maschinenlesbaren Kennzeichnung handelt es sich vorzugsweise um ein Barcode-System, welches mit Hilfe geeigneter Aufkleber auf den Prüftafeln aufgebracht werden kann.

Eine derartige Kennzeichnung der Prüftafeln hat den Vorteil, daß unmittelbar mit der Prüftafel selbst alle relevanten Informationen verknüpft sind. Es kann daher nicht mehr zu Verwechselungen kommen, bei denen z.B. eine Prüftafel falsch vermessen wird oder die gewonnenen Meßergebnisse einer anderen Prüftafel zugeordnet werden. Ferner muß innerhalb der Aufgabestation nicht streng auf die Reihenfolge der Prüftafeln geachtet werden, da die Prüftafeln nicht durch ihre Position innerhalb der Reihe der zu vermessenden Tafel identifiziert werden. Diese Flexibilität erlaubt es insbesondere auch, daß verschiedene Benutzer Prüftafeln erzeugen und dem Meßverfahren zuführen. Jeder einzelne Benutzer muß seine Prüftafel nur mit den entsprechenden Kennzeichnungen versehen und braucht sich nicht um bestimmte Regeln bei der Ablage der Prüftafeln zu kümmern. Ebenso kann bei der Ausgabestation eine automatische Zuordnung der Prüftafeln zu verschiedenen Benutzern der Meßanlage erfolgen. Schließlich kann die Kennzeichnung auch Information über die Form und Größe der Prüftafel enthalten. Dies erleichtert die Bearbeitung verschiedener Prüftafeltypen, da ihre Form/Größe nicht mehr durch sonstige Sensoren festgestellt werden muß.

Das erfindungsgemäße Verfahren wird vorzugsweise eingesetzt, um Schichtdicke, Verlauf, Farbton, die Farbkoordinaten L (Helligkeit), a (Rot-Grün). b (Gelb-Blau), Härte, Haze und/oder Glanz der Prüftafeln zu vermessen. Es ist jedoch auch möglich, andere Eigenschaften (z.B. Ritzfestigkeit, Klebfreiheit Benetzungsstabilität, Oberflächen-Topographie.) zu vermessen. Zu diesem Zweck muß lediglich ein entsprechendes Meßgerät zur Verfügung gestellt werden, welches von der Greif- und Bewegungsvorrichtung bedient werden kann.
Die Bestimmung von Farbeigenschaften ist dabei von besonderer Bedeutung. Für manche Prüfaufgaben sind detaillierte und umfangreiche Informationen über die Farbe erforderlich, z.B. die Bestimmung der Wolkenbildung bei Metalliclacken oder die Bestimmung der Farbtonstabilität von Decklacken. Für solche Meßaufgaben ist es von Vorteil, ein Farbmeßgerät zu benutzen, welches die Farbkoordinaten L, a, b liefert. Ganz besonders vorteilhaft ist es, wenn diese Farbwerte in unterschiedlichen Beobachtungswinkeln ermittelt werden, vorzugsweise unter 20°-30°, 40°-50° und 70°-80°, ganz besonders bevorzugt unter 25°, 45° und 75°.

Aus den verschiedenartigen Meßgrößen, die mit dem erfindungsgemäßen Verfahren gewonnen werden, können weiterhin durch Korrelation bzw. die Beachtung des funktionalen Zusammenhanges völlig neue Eigenschaften ermittelt werden, z.B. das Deckvermögen oder die Wolkenbildung in der Schrägsicht unter 75°.

Damit die im erfindungsgemäßen Verfahren eingesetzten Meßgeräte präzise Daten liefern, ist es erforderlich, sie in gewissen Zeitabständen einer Prüfmittelüberwachung/kalibrierung zu unterziehen. Dabei erweist es sich als problematisch, daß die Meßgeräte ausgebaut und quasi off-line kalibriert werden müssen. Dies ist umständlich, kostet Zeit und ist fehlerbehaftet, da der Vorgang auch vergessen werden kann.
Zum erfindungsgemäßen Verfahren gehört daher auch eine automatische Kalibrierung der Meßgeräte. Dabei sorgt eine entsprechende Programmierung der Greif- und Bewegungsvorrichtung dafür, daß diese die vorgegebenen Kalibrierungen in bezug auf Termin, Art der Kalibrierung und Dokumentation selbständig durchführt. Die Greif- und Bewegungsvorrichtung hat zu diesem Zweck jederzeit Zugriff auf die Standards, die sie für die Kalibrierung der Meßgeräte benötigt und die in ihrem Wirkungskreis angeordnet sind. Die Kalibrierung kann in der Regel so erfolgen, daß mit Hilfe des Standards (anstelle der Prüftafel) eine Referenzmessung erfolgt.

Zur Erfindung gehört auch eine Vorrichtung zur Vermessung von lackierten Prüftafeln gemäß des erfindungsgemäßen Verfahrens, welche folgende Elemente enthält:
a) eine Aufgabestation für die zu vermessenden Prüftafeln,
b) eine Greif- und Bewegungsvorrichtung für die Bewegung der Prüftafeln und ihre Vermessung mit Meßgeräten,
c) mindestens ein Meßgerät, mit welchem die interessierenden Beschichtungseigenschaften der Prüftafel gemessen werden können,
d) eine Ablagestation für die vermessenen Prüftafeln.

Mit der erfindungsgemäßen Vorrichtung kann insbesondere das oben geschilderte erfindungsgemäße Verfahren durchgeführt werden. Ein Kernstück hierbei ist die Greif- und Bewegungsvorrichtung (Manipulator), mit Hilfe derer der gesamte Meßvorgang vollautomatisch ablaufen kann. Bei einer derartigen Vorrichtung kann es sich insbesondere auch um einen Labor-Roboter handeln, d.h. eine Vorrichtung, die fest installiert ist, einen Greifarm mit mindestens einem Gelenk und einer Greifvorrichtung hat und die einprogrammierte oder vorgeführte Bewegungsabläufe durchführen kann. Mit einer derartigen automatischen Vermessung der Prüftafeln kann nicht nur der personelle Aufwand erheblich verringert werden, sondern auch eine höhere Präzision und Verläßlichkeit der Meßergebnisse erzielt werden. Dies geschieht zum einen dadurch, daß problemlos mehrere Messungen derselben Eigenschaft gemacht werden können, die dann statistisch ausgewertet werden können, zum anderen dadurch, daß die jeweiligen Messungen genau mit einem Meßort korreliert sind und somit verläßlicher sind und auch untereinander abgeglichen werden können.

Desweiteren enthält die erfindungsgemäße Vorrichtung vorzugsweise ein Lesegerät für die elektronische Erfassung von Kennzeichnungsdaten von Prüftafeln. Hierbei kann es sich vorzugsweise um ein Barcode-Lesegerät handeln. Die Erfassung des Barcodes kann derart erfolgen, daß die Prüftafel mit dem Barcode-Aufkleber an dem Lesegerät vorbeigeführt wird, oder das umgekehrt die Greif- und Bewegevorrichtung das Lesegerät ergreift und an der ruhenden Prüftafel vorbeiführt. Bei der letzten Variante läuft die Erfassung des Barcodes im Prinzip ähnlich ab wie die Messung der übrigen Beschichtungseigenschaften. Umgekehrt ist es im Rahmen der Erfindung auch für die Messungen denkbar, daß die Prüftafel an den ruhenden Meßgeräten entlanggeführt wird und dabei die Messung erfolgt.

Innerhalb der erfindungsgemäßen Vorrichtung sind die Meßgeräte und/oder das ggfs. vorhandene Lesegerät der Kennzeichnung mit einer elektronischen Datenverarbeitung verbunden. Hierbei kann es sich z.B. um einen üblichen Personal-Computer PC handeln, welcher entsprechende Elektronik für die Erfassung und ggfs. Digitalisierung von Meßdaten hat. Durch eine derartige elektronische Erfassung wird die Automatisierung der Vorrichtung abgerundet. Die gewonnenen Meßwerte werden unmittelbar in der elektronischen Zentraleinheit gespeichert und ausgewertet. Ferner kann diese elektronische Zentraleinheit auch die Steuerung der Greif- und Bewegungsvorrichtung übernehmen. Dies ist besonders dann zweckmäßig, wenn sie die elektronisch erfaßten Kennzeichnungsdaten der Prüftafel erhält und in Abhängigkeit hiervon das anzuwendende Meßprogramm (zu ergreifende Meßgeräte, anzufahrende Positionen auf der Prüftafel....) an die Greif- und Bewegungsvorrichtung weiterleitet.

Vorzugsweise sind die Aufgabestation und/oder Ablagestation innerhalb der erfindungsgemäßen Vorrichtung als Magazine für die Prüftafeln ausgestaltet. Derartige Magazine können außerhalb der Vorrichtung mit Prüftafeln bestückt werden und sorgen dann innerhalb der Vorrichtung dafür, daß die Prüftafeln an definierten Orten stehen und somit für den automatischen Greifer leicht und sicher aufzufinden sind. Femer können verschiedene Einheiten des Labors jeweils eigene Magazine haben und befüllen, so daß die Zuordnung der Prüftafel organisatorisch optimiert ist.
Die Magazine können dabei insbesondere so ausgebildet sein, daß sie Prüftafeln unterschiedlicher Größe und Form aufnehmen können. In der Steuerung der Greif- und Bewegungsvorrichtung sind dabei die unterschiedlichen Meßprogramme, zugeschnitten auf unterschiedliche Tafelgrößen und -goemetrien, hinterlegt

Innerhalb der erfindungsgemäßen Vorrichtung können verschiedene Meßgeräte eingesetzt werden. Bedingung ist lediglich, daß diese von der Greif- und Bewegungsvorrichtung bewegt und bedient werden können. Dabei ist es insbesondere auch möglich, die Meßgeräte an standardisierten Plätzen bereit zu halten, so daß sie leicht gegeneinander ausgetauscht werden können, oder daß neue Meßgeräte in die Vorrichtung eingefügt werden können. In diesem Falle ist lediglich eine Abstimmung der Greif- und Bewegevorrichtung erforderlich, daß diese ein neues Gerät auch findet und korrekt bedient. Im Rahmen der erfindungsgemäßen Vorrichtung anwendbar sind insbesondere Meßgeräte für Schichtdicke, Verlauf, Farbton, Haze und Glanz sowie zur Abtastung der Topographie (Perthometer) der Prüftafeln. Die Vorrichtung ist jedoch, wie bereits erwähnt, nicht auf diese Meßgeräte beschränkt.

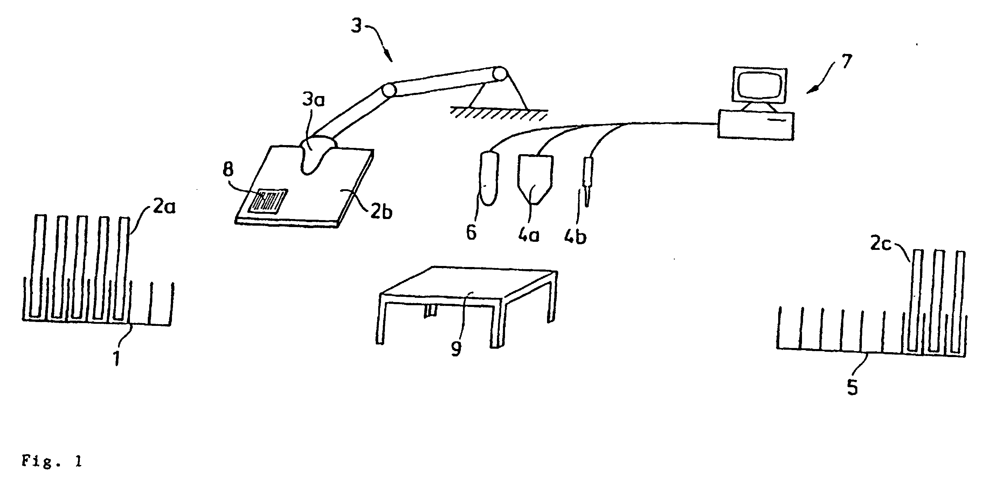

Im folgenden wird die Erfindung mit Hilfe der Abbildung beispielhaft erläutert.

Die Abbildung zeigt eine Realisierung der erfindungsgemäßen Vorrichtung mit verschiedenen Elementen. Die lackierten Prüftafeln 2 werden nach ihrer Herstellung zunächst in der Aufgabestation 1 abgelegt. In der Abbildung ist die Aufgabestation 1 als ein Magazin realisiert, in welches die Prüftafeln 2a eingesetzt sind. Eine Greif- und Bewegungsvorrichtung 3 greift dann die Prüftafeln 2a aus dem Magazin 1, um sie der Messung zuzuführen. Die Greifund Bewegungsvorrichtung kann insbesondere als ein Labor-Roboter-System 3 ausgestaltet sein, z.B. ein 5-Achsen Roboter (wie z.B. "MOVEMASTER RV-E2/E3J" von Mitsubishi Electric, Ratingen). Die mit Hilfe des Greifers 3a gehaltene Prüftafel 2b wird sodann von der Aufnahmestation 1 zu einer Meßstation 9 überführt. Bei der Meßstation 9 kann es sich insbesondere um einen Meßtisch handeln, auf dem die Prüftafel ausreichend fixiert und ausgerichtet ist. Eine Fixierung der Prüftafel kann dabei durch geeignete Anschläge am Rand des Tisches erfolgen, oder durch ausreichende Reibungskräfte zwischen Unterlage und Probe oder mit einer Vakuumtechnischen Haltevorrichtung (Sauger).

Die Prüftafeln 2 enthalten ferner einen Aufkleber mit einem Barcode 8. Durch diesen Barcode (denkbar sind auch andere Codierungsarten, sofern sie maschinenlesbar sind) werden wichtige Informationen über die Prüftafel wiedergegeben. Hierbei kann es sich z.B. um Hersteller der Tafel, Herstellungsverfahren, Beschichtungsmaterial, gewünschte Messung, Orte der Messung usw. handeln. Der Barcode-Aufkleber 8 kann mit Hilfe eines automatischen Lesegerätes 6 erkannt werden.
Die auf der Meßstation 9 befindlichen Prüftafeln werden schließlich auf die gewünschten Eigenschaften hin vermessen. Hierzu ergreift der Roboter 3 die Meßgeräte 4a,4b nacheinander und führt sie an die gewünschten Stellen der Prüftafel 2. Beispiele für geeignete Meßgeräte sind magnet-induktive oder Wirbelstrom Schichtdickenmeßgeräte (z.B. MINITEST Geräte der Firma Elektro-Physik, Köln), Winkelspektrometer (z.B. X-Rite® MA68) oder Glanz-Meßgeräte (z.B. micro-TRI-gloss, wave-scan plus oder mirror-TRI-gloss der Firma BYK-Gardner, Geretsried) Perthometer der Fa. Mahr. Die genauen Meßorte können dabei von Tafel zu Tafel variieren. Durch mehrfache Messung an einem Ort oder durch Messungen an verschiedenen Orten kann eine umfangreichere Information über die Beschichtung gewonnen werden, als dies bei dem üblichen manuellen Meßverfahren möglich ist. Insbesondere ist es damit auch möglich, die Messungen verschiedener Eigenschaften untereinander zu korrelieren. Die Meßgeräte 4 und das Lesegerät 6 sind über entsprechende Leitungen mit einer zentralen Datenerfassungseinheit, einem Personal-Computer 7, verbunden. Die Daten können dabei entweder noch innerhalb des Meßgerätes oder innerhalb des PCs 7 digitalisiert werden.

Nach Abschluß der Messungen ergreift der Roboter 3 die Prüftafel auf der Meßstation und überführt diese in die Ablagestation 5. Hierbei kann es sich wieder um ein Magazin handeln, in welches die Prüftafeln der Reihe nach eingesetzt werden. Ebenso ist es möglich, mehrere Magazine parallel nebeneinander vorzusehen, wobei der Roboter 3 entsprechend den Informationen auf der Codierung 8 eine Zuordnung der Prüftafeln in die verschiedenen Magazine vornimmt.

In Versuchen wurde das erfindungsgemäße Verfahren zur Beurteilung der Wolkenbildung eingesetzt. Verglichen wurde ein Lack, der zu Wolkenbildung neigt, mit einem Lack, der keine Wolkenbildung zeigt. Gemessen wurde jeweils die Helligkeit des Lackfilms bei unterschiedlichen Schichtdicken anhand eines keilförmig applizierten Basislackfilms (5-20 µm). Die Auswertung in bezug auf die Verläufe der Helligkeit bei 25° Beobachtungswinkel zeigte keine Unterschiede der Formulierungen, wie sie jedoch visuell deutlich wahrgenommen werden. Erst eine Änderung des Beobachtungswinkels auf 45° und/oder 75° zeigt die deutlichen Unterschiede der Formulierungen. Dieses Ergebnis belegt die Notwendigkeit einer vollständigen Meßdatenbestimmung, z.B. wie im dargestellten Versuch unter verschiedenen Beobachtungswinkeln. Mit dem erfindungsgemäßen, vollautomatisch arbeitenden Verfahren ist dies schnell und präzise möglich, während bei den herkömmlichen manuellen Meßmethoden aus Zeit- und Kostengründen nur ein unzureichendes Minimalprogramm ausgeführt werden konnte.

In der Praxis des Lackierens (z.B. von Automobilkarossen) ist es eine übliche Aufgabe, die Auswirkung unterschiedlicher Lackierparameter und Lackierungsprozesse auf den Farbton zu ermitteln. Versuche haben auch hier gezeigt, daß Unterschiede oft durch den Wert L in 25° Geometrie nicht detektiert wurden, jedoch in anderen Geometrien der Beobachtung und insbesondere durch die Farbkoordinaten a bzw. b.

## Patentansprüche

1. Verfahren zur Vermessung von lackierten Prüftafeln (2), enthaltend folgende Schritte:
a) Ablage der Prüftafeln (2a) in einer Aufgabestation (1),
b) Überführung einer Prüftafel (2b) aus der Aufgabestation (1) zu einer Meßstation (9) durch eine Greif- und Bewegungsvorrichtung (3),
c) Ergreifen der Meßgeräte (4) durch die Greif- und Bewegungsvorrichtung (3) und Durchführung der Messungen an der Prüftafel (2b) in der Meßstation (9),
d) Überführung der vermessenen Prüftafel (2c) in die Ablagestation (5) durch die Greif- und Bewegungsvorrichtung (3).

2. Verfahren nach Anspruch 1,
**dadurch gekennzeichnet, daß** die an der Prüftafel (2b) gewonnenen Meßdaten elektronisch erfaßt, gespeichert, ausgewertet und/oder dargestellt werden.

3. Verfahren nach einem der Ansprüche 1 oder 2,
**dadurch gekennzeichnet, daß**
a) die Prüftafeln (2) mit einer maschinenlesbaren Kennzeichnung (8), vorzugsweise einem Barcode, über die gewünschte Art der Messung, die Orte der Messung und/oder die Identifizierung der Prüftafel versehen sind,
b) die maschinenlesbare Kennzeichnung (8) erfaßt wird und die darin enthaltenen Informationen in die Steuerungsvorrichtung der Greif- und Bewegungsvorrichtung (3) und/oder die Datenerfassung (7) übernommen werden.

4. Verfahren nach einem der Ansprüche 1 bis 3,
**dadurch gekennzeichnet, daß** hiermit Schichtdicke, Verlauf, Farbton, die Farbkoordinaten L, a, b, Haze und/oder Glanz der Prüftafeln (2) sowie die Toptgraphie gemessenwerden.

5. Verfahren nach einem der Ansprüche 1 bis 4,
**dadurch gekennzeichnet, daß** die Farbkoordinaten L, a, b unter verschiedenen Beobachtungswinkeln ermittelt werden, vorzugsweise unter 20°-30°, 40°-50° und 70°-80°, ganz besonders bevorzugt unter 25°, 45° und 75°.

6. Verfahren nach einem der Ansprüche 1 bis 5,
**dadurch gekennzeichnet, daß** ortsaufgelöst gemessen wird und/oder die Meßwerte mit dem Meßort auf der Prüftafel (2) korreliert werden.

7. Verfahren nach einem der Ansprüche 1 bis 6,
**dadurch gekennzeichnet, daß** die Meßwerte entlang von Bahnen oder in Flächenbereichen auf den Prüftafeln ermittelt werden.

8. Verfahren nach einem der Ansprüche 1 bis 7,
**dadurch gekennzeichnet, daß** verschiedene Meßwerte miteinander korreliert werden.

9. Verfahren nach einem der Ansprüche 1 bis 8,
**dadurch gekennzeichnet, daß** mit im Wirkungsbereich der Greif- und Bewegungsvorrichtung (3) angeordneten Standards eine Kalibrierung der Meßgeräte (4) vorgenommen wird.

10. Verfahren nach Anspruch 9,
**dadurch gekennzeichnet, daß** die Kalibrierung durch eine mit dem Standard anstelle der lackierten Prüftafel durchgeführte Referenzmessung erfolgt.

11. Vorrichtung zur Vermessung von lackierten Prüftafeln (2) in einem Verfahren nach einem der Ansprüche 1-10, enthaltend
a) eine Aufgabestation (1) für die zu vermessenden Prüftafeln (2a),
b) eine Greif- und Bewegungsvorrichtung (3) für die Bewegung der Prüftafeln (2) und deren Vermessung mit den Meßgeräten (4),
c) mindestens ein Meßgerät (4) für die Messung interessierender Beschichtungseigenschaften der Prüftafeln (2),
d) eine Ablagestation (5) für die vermessenen Prüftafeln (2c).

12. Vorrichtung nach Anspruch 11,
**dadurch gekennzeichnet, daß** sie ein Lesegerät (6) für die elektronische Erfassung von Kennzeichnungsdaten der Prüftafel (2b), vorzugsweise ein Barcode-Lesegerät, enthält.

13. Vorrichtung nach einem der Ansprüche 11 oder 12,
**dadurch gekennzeichnet, daß** die Greif- oder Bewegungsvorrichtung (3), die Meßgeräte (4) und/oder das Lesegerät (6) mit einer elektronischen Datenverarbeitung (7) verbunden sind.

14. Vorrichtung nach einem der Ansprüche 11 bis 13,
**dadurch gekennzeichnet, daß** die Aufgabestation (1) und/oder die Ablagestation (5) als Magazine für die Prüftafeln (2a,2c) ausgebildet sind.

15. Vorrichtung nach einem der Ansprüche 11 bis 14,
**dadurch gekennzeichnet, daß** sie Meßgeräte (4a,4b) für Schichtdicke, Verlauf, Farbton, Haze und/oder Glanz der Prüftafeln (2) enthält.

## Claims

1. Method for measuring coated test panels (2), comprising the following steps:
a) depositing the test panels (2a) in a feed station (1),
b) transferring a test panel (2b) from the feed station (1) to a measuring station (9) by means of a gripping and moving device (3),
c) gripping the measuring devices (4) by means of the gripping and moving device (3) and carrying out the measurements on the test panel (2b) in the measuring station (9),
d) transferring the measured test panel (2c) into the delivery station (5) by means of the gripping and moving device (3).

2. Method according to claim 1, **characterized in that** the measurement data obtained on the test panel (2b) are recorded, stored, evaluated and/or displayed electronically.

3. Method according to either of claims 1 and 2, **characterized in that**
a) the test panels (2) are provided with a machine-readable identifier (8), preferably a barcode, relating to the desired type of measurement, the locations of the measurement and/or the identification of the test panel,
b) the machine-readable identifier (8) is recorded and the information contained therein is accepted into the control device of the gripping and moving device (3) and/or the data acquisition system (7).

4. Method according to one of claims 1 to 3, **characterized in that** film thickness, leveling, color, the color coordinates L, a, b, haze and/or gloss of the test panels (2), as well as the topography, are measured herewith.

5. Method according to one of claims 1 to 4, **characterized in that** the color coordinates L, a, b are ascertained at various angles of observation, preferably at 20°-30°, 40°-50° and 70°-80°, very particularly preferably at 25°, 45° and 75°.

6. Method according to one of claims 1 to 5, **characterized in that** spatially-resolved measurements are carried out and/or the measured values are correlated with the measurement location on the test panel (2).

7. Method according to one of claims 1 to 6, **characterized in that** the measured values are ascertained along paths or in area regions on the test panels.

8. Method according to one of claims 1 to 7, **characterized in that** various measured values are correlated with one another.

9. Method according to one of claims 1 to 8, **characterized in that** a calibration of the measuring devices (4) is undertaken using standards arranged in the range of action of the gripping and moving device (3).

10. Method according to claim 9, **characterized in that** the calibration is performed by means of a reference measurement carried out using the standard instead of the coated test panel.

11. Apparatus for measuring coated test panels (2) in a method according to one of claims 1-10, containing
a) a feed station (1) for the test panels (2a) to be measured,
b) a gripping and moving device (3) for the movement of the test panels (2) and their measurement using the measuring devices (4),
c) at least one measuring device (4) for the measurement of coating properties of interest of the test panels (2),
d) a delivery station (5) for the measured test panels (2c).

12. Apparatus according to claim 11, **characterized in that** it contains a reading device (6) for the electronic recording of identifying data from the test panel (2b), preferably a barcode reader.

13. Apparatus according to either of claims 11 and 12, **characterized in that** the gripping or moving device (3), the measuring devices (4) and/or the reading device (6) are connected to an electronic data processing system (7).

14. Apparatus according to one of claims 11 to 13, **characterized in that** the feed station (1) and/or the delivery station (5) are designed as magazines for the test panels (2a, 2c).

15. Apparatus according to one of claims 11 to 14, **characterized in that** it contains measuring devices (4a, 4b) for film thickness, leveling, color, haze and/or gloss of the test panels (2).

## Revendications

1. Procédé de mesure de plaques de test peintes (2), comportant les étapes suivantes:
a) dépôt des plaques de test (2a) dans une station de chargement (1),
b) transfert d'une plaque de test (2b) de la station de chargement (1) à une station de mesure (9) par un dispositif de saisie et de déplacement (3),
c) saisie des appareils de mesure (4) par le dispositif de saisie et de déplacement (3) et exécution des mesures sur la plaque de test (2b) dans la station de mesure (9),
d) transfert de la plaque de test mesurée (2c) dans la station de déchargement (5) par le dispositif de saisie et de déplacement (3).

2. Procédé suivant la revendication 1, **caractérisé en ce que** les données de mesure obtenues sur la plaque de test (2b) sont saisies, mémorisées, exploitées et/ou représentées par voie électronique.

3. Procédé suivant l'une des revendications 1 ou 2, **caractérisé en ce que**
a) les plaques de test (2) sont pourvues d'un marquage (8) lisible par une machine, de préférence un code-barres, concernant le type de mesure souhaité, les endroits de mesure et/ou l'identification de la plaque de test,
b) le marquage lisible par une machine (8) est saisi et les informations qu'il contient sont reprises dans le dispositif de commande du dispositif de saisie et de déplacement (3) et/ou dans la saisie de données (7).

4. Procédé suivant l'une des revendications 1 à 3, **caractérisé en ce que** l'on mesure par celui-ci l'épaisseur de couche, le dégradé, la nuance, les coordonnées de la couleur L, a, b, le voile et/ou le lustre des plaques de test (2), ainsi que la topographie.

5. Procédé suivant l'une des revendications 1 à 4, **caractérisé en ce que** l'on détermine les coordonnées de la couleur L, a, b sous différents angles d'observation, de préférence sous 20°-30°, 40°-50° et 70°-80°, et en préférant particulièrement 25°, 45° et 75°.

6. Procédé suivant l'une des revendications 1 à 5, **caractérisé en ce que** l'on effectue des mesures résolues localement et/ou on établit une corrélation entre les valeurs de mesure et l'endroit de mesure sur la plaque de test (2).

7. Procédé suivant l'une des revendications 1 à 6, **caractérisé en ce que** les valeurs de mesure sont déterminées le long de trajectoires ou dans des zones superficielles sur les plaques de test.

8. Procédé suivant l'une des revendications 1 à 7, **caractérisé en ce que** différentes valeurs de mesure sont corrélées les unes avec les autres.

9. Procédé suivant l'une des revendications 1 à 8, **caractérisé en ce que** l'on opère un calibrage des appareils de mesure (4) avec des étalons disposés dans la zone d'action du dispositif de saisie et de déplacement (3).

10. Procédé suivant la revendication 9, **caractérisé en ce que** le calibrage est effectué par une mesure de référence exécutée avec l'étalon au lieu de la plaque de test peinte.

11. Dispositif de mesure de plaques de test peintes (2) par un procédé suivant l'une des revendications 1 à 10, comprenant
a) une station de chargement (1) pour les plaques de test à mesurer (2a),
b) un dispositif de saisie et de déplacement (3) pour le déplacement des plaques de test (2) et leur mesure avec les appareils de mesure (4),
c) au moins un appareil de mesure (4) pour la mesure de propriétés intéressantes du revêtement des plaques de test (2),
d) une station de déchargement (5) pour les plaques de test mesurées (2c).

12. Dispositif suivant la revendication 11, **caractérisé en ce qu'**il comprend un appareil de lecture (6) pour la saisie électronique de données de marquage de la plaque de test (2b), de préférence un lecteur de codes-barres.

13. Dispositif suivant l'une des revendications 11 ou 12, **caractérisé en ce que** le dispositif de saisie et de déplacement (3), les appareils de mesure (4) et/ou le lecteur (6) sont raccordés à un dispositif électronique de traitement de données (7).

14. Dispositif suivant l'une des revendications 11 à 13, **caractérisé en ce que** la station de chargement (1) et/ou la station de déchargement (5) se présentent sous la forme de magasins pour les plaques de test (2a, 2c).

15. Dispositif suivant l'une des revendications 11 à 14, **caractérisé en ce qu'**il comporte des appareils de mesure (4a, 4b) pour l'épaisseur de couche, le dégradé, la nuance, le voile et/ou le lustre des plaques de test (2).
